Europäisches Patentamt

(19) European Patent Office

Office européen des brevets

(11) Publication number: **0 366 673 B1**

# EUROPEAN PATENT SPECIFICATION

(12)

(45) Date of publication of patent specification: 16.09.92  (51) Int. Cl.⁵: **G01N 33/543**, G01N 33/536, G01N 33/569

(21) Application number: **88904993.8**

(22) Date of filing: **08.06.88**

(86) International application number:
**PCT/GB88/00446**

(87) International publication number:
**WO 88/09933 (15.12.88 88/27)**

(54) **IMMUNOASSAY METHOD.**

(30) Priority: **09.06.87 GB 8713418**
**17.06.87 GB 8714213**

(43) Date of publication of application:
**09.05.90 Bulletin 90/19**

(45) Publication of the grant of the patent:
**16.09.92 Bulletin 92/38**

(84) Designated Contracting States:
**AT BE CH DE FR GB IT LI LU NL SE**

(56) References cited:
**EP-A- 161 107**
**WO-A-86/04993**
**GB-A- 2 029 011**
**US-A- 4 067 959**
**US-A- 4 189 466**

**JOURNAL OF IMMUNOLOGICAL METHODS,
vol.88, no.1, 1986, Elsevier, Amsterdam (NL);
H.SCHMITZ et al., pp.115-120**

(73) Proprietor: **CAMBRIDGE LIFE SCIENCES PLC
Cambridgeshire Business Park Angel Drove
Ely Cambridgeshire CB7 4DT(GB)**

(72) Inventor: **WALKER, Michael Strachan, c/o
Walker Lab. Ltd.
Cambridgeshire Business Park, Angel Drove
Ely, Cambridgeshire CB7 4DT(GB)**
Inventor: **CASBURN-BUDD, Roger Henry
3 Fordham Road
Isleham, Cambridgeshire CB5 8OU(GB)**

(74) Representative: **Perry, Robert Edward et al
GILL JENNINGS & EVERY 53-64 Chancery
Lane
London WC2A 1HN(GB)**

## Description

### Field of the Invention

This invention relates to assay systems for the detection of antibodies of all immunoglobulin classes in biological fluids.

### Background of the Invention

Mammals, including humans, produce antibodies to invading organisms such as bacteria or viruses. In some diseases, e.g. rheumatoid arthritis, antibodies against the animals own tissues may be produced; these are known as auto-antibodies. The detection of antibodies in serum of patients (human or veterinary) is an important diagnostic procedure in the investigation of infectious diseases, auto-immune diseases, and tolerance to therapy with drug preparations of animal origin, e.g. beef insulin or mouse monoclonal antibodies.

Antibodies are multi-valent, in that they can bind more than one molecule of their specific antigen. This principle has been widely used to detect antibodies (mainly IgM class) by agglutination techniques using red blood cells coated with antigen. The antibodies cause cells to clump or agglutinate together.

Antibodies are also detected by solid phase immunoassays, in which a specific antigen is coated on to a solid phase and then allowed to react with a serum sample suspected of having antibodies present in it. Antibodies which bind to the solid phase may then be detected using a labelled anti-species IgG antibody (e.g. anti-human IgG for patient's serum). The label may be, for example, an isotopic or non-isotopic material, e.g. horseradish peroxidase in the case of an enzymatic label. Enzyme activity detected in the final reaction is proportional to the amount of antibody in the patient's serum. By referring to the activity in pre-calibrated standard sera, the antibody content of unknown samples may be quantitated.

In the case of anti-microbial antibodies, IgM antibodies occur in the early stages of the disease and IgG in later stages. Using current technology, two different assays are normally employed to detect antibodies in early and late infection stages.

In AIDS testing, current methods detect mainly IgG antibodies and can miss IgM antibodies. Therefore, recent AIDS infections may be missed unless a specific IgM assay is employed.

An object of the present invention is to reduce the complexity and to minimise the disadvantages of existing methods and to produce an immunoassay for the detection of antibodies which is simple, convenient and able to detect all classes of immuno-globulins simultaneously, to ensure that pathological changes in immune responses are detected in their early stages.

### Summary of the Invention

According to the present invention, a method for the detection of antibodies suspected of being present in a specimen comprises:

a) mixing the specimen and labelled purified antigen with the same purified antigen attached to a solid phase support, to form an immobilised labelled antigen-antibody-attached antigen complex;

b) washing away any labelled antigen which is not part of the complex; and

c) determining the activity of labelled antigen present on the solid phase.

The antigen that is labelled and the antigen that is attached to the solid phase are the "same" in the sense that they have the same antigenic site.

The present invention is based on the realisation that the antigens of multi-valent antibodies will not form complexes in the absence of their specific antibodies.

Detection of such antibodies in biological fluids can be achieved in a simple one-step reaction. Clear and accurate results may be achieved.

All classes of antibodies are detected. An additional advantage is that antibodies from the sera of different animal species may be detected by the same assay, provided that they have a common antigen.

### Description of the Invention

The present invention involves coating a solid phase support (which may be polystyrene TSP pins or microwells or polystyrene or PVC beads or slides) with purified antigen, any unoccupied binding sites being blocked with inert proteins.

Diluted sera are then mixed with a solution of purified antigen conjugated to a "label". The label may be an isotope such as $^{125}$I or an enzyme such as horseradish peroxidase or a fluorescent or chemiluminescent label.

Once in contact with the antibodies, the labelled and solid phase antigens form immobilised complexes which remain in contact with the solid phase. The period of contact is related to individual assays. Labelled antigen which is not specifically bound to the solid phase is washed away by a buffer solution.

The amount of labelled antigen bound to the solid phase is determined (e.g. by gamma counting or by incubation with enzyme substrate in the case of non-isotopic systems). By reference to a standard curve of known amounts of antibody, the unknown samples may be quantified.

The antibodies may be, for example, rheumatoid factors or antibodies to HIV; the process of the invention may then be used to test for, or monitor, rheumatoid arthritis or AIDS in a subject. Rheumatoid factors are anti-globulins and therefore the antigen which is then used in the process of the invention will be a globulin, e.g. IgG.

The following Example illustrates the invention.

Example

Human IgG antibody is coated on to the tips of NUNC TSP pins on a plate, at a concentration of 10 μg/ml in a carbonate buffer comprising 0.1 M carbonate, pH 9.6, and incubated overnight at room temperature. Pins are then blocked with a solution containing 0.5% Bovine Serum Albumin, 5% Lactose and 0.1% Azide, and then dried. Plates can be stored for six months at 4°C.

Next, a plate with 'U' wells is filled with a 1:100 dilution of patients serum, to 50 μl in each well, followed by 50 μl of enzyme-labelled human IgG into all the filled wells. The enzyme is peroxidase. The pins are inserted into the wells and the whole system is incubated for ten minutes at room temperature.

The pins are then washed and placed into a flat bottom tray containing 200 μl of a substrate solution comprising o-phenylenediamine in HCl in citrate buffer. They are incubated at room temperature for a further ten minutes. Colour development is stopped by the addition of 4M sulphuric acid.

Claims

1. A method for detecting antibodies suspected of being present in a specimen, which comprises:
   a) mixing the specimen and labelled purified antigen with the same purified antigen attached to a solid phase support, to form an immobilised labelled antigen-antibody-attached antigen complex;
   b) washing away any labelled antigen which is not part of the complex; and
   c) determining the activity of labelled antigen present on the solid phase.

2. A method according to claim 1, in which the antibodies are rheumatoid factors.

3. A method according to claim 1, in which the antibodies are antibodies to HIV.

Patentansprüche

1. Verfahren zum Nachweis von in einer Probe vermuteten Antikörpern umfassend:
   a) Mischen der Probe und des markierten gereinigten Antigens mit dem gleichen, an einen Festphasenträger gebundenen, gereinigten Antigen, um einen immobilisierten Komplex von markiertem Antigen, Antikörper und gebundenem Antigen zu bilden;
   b) Wegwaschen des Anteils von markiertem Antigen, das nicht komplexgebunden ist;
   c) Bestimmung der Aktivität des auf der Festphase befindlichen Antigens.
   Das markierte Antigen und das an die Festphase gekoppelte Antigen sind in dem Sinne "gleich", als sie die gleiche antigenische Bindungsstelle aufweisen.

2. Verfahren nach Anspruch 1, bei welchem die Antikörper Rheumatoidfaktoren sind.

3. Verfahren nach Anspruch 1, bei welchem die Antikörper Antikörper gegen HIV sind.

Revendications

1. Procédé de détection des anticorps supposés être présents dans un échantillon, comprenant :
   a) le mélange de l'échantillon et d'un antigène purifié marqué avec le même antigène purifié fixé à un support en phase solide pour former un complexe antigène marqué-anticorps-antigène fixé immobilisé ;
   b) l'élimination par lavage de tout antigène marqué qui ne fait pas partie du complexe ; et
   c) la détermination de l'activité de l'antigène marqué présent sur la phase solide.

2. Procédé selon la revendication 1, dans lequel les anticorps sont des facteurs rhumatoïdes.

3. Procédé selon la revendication 1, dans lequel les anticorps sont des anticorps contre HIV.